# EUROPEAN PATENT APPLICATION

(11) **EP 4 068 293 A1**
(43) Date of publication of application: **05.10.2022**
(21) Application number: 21382272.9
(22) Date of filing: 31.03.2021
(51) Int. Cl.: G16H 10/40, G16H 40/40

(54) **METHOD FOR PERFORMING A MAINTENANCE TASK IN A LABORATORY SYSTEM AND LABORATORY SYSTEM**

(71) Applicant: Roche Diagnostics GmbH, 68305 Mannheim (DE); F. Hoffmann-La Roche AG, 4070 Basel (CH)
(72) Inventor: Duquez, Ruben, 08174 Barcelona (ES)
(74) Representative: Bittner, Thomas L.

(57) **Abstract**

A method for performing a maintenance task in a laboratory system configured for at least one of sample pre-analytics and sample analytics is disclosed, the method comprising: providing first permission data in a memory (22) accessible by a maintenance access control device (21) assigned to a laboratory system (20) configured for at least one of sample pre-analytics and sample analytics, the first permission data being indicative of a plurality of user-specific maintenance modes applicable in the laboratory system (20), wherein each of the plurality of user-specific maintenance modes is assigned to at least one user and indicating one or more maintenance tasks permitted to be conducted by the at least one user in the user-specific maintenance mode; receiving user identification data indicative of a user in the maintenance access control device (21); receiving user selection data indicative of a user selection of a first maintenance mode from the plurality of user-specific maintenance modes in the maintenance access control device (21); and determining whether the first maintenance mode is assigned to the user by the maintenance access control device (21). Access control data are provided in the maintenance access control device (21), the access control data is indicative of one of permitting the user to conduct the first maintenance mode, if is determined that the first maintenance mode is assigned to the user; and not permitting the user to conduct the first maintenance mode, if is determined that the first maintenance mode is not assigned to the user. Operation of the laboratory system (20) is controlled according to the access control data. Furthermore, a laboratory system is provided.

## Description

The present invention refers to a method for performing a maintenance task in a laboratory system configured for at least one of sample pre-analytics and sample analytics and a laboratory system.

### Background

Laboratory systems may be configured to be applied for at least one of sample pre-analytics and sample analytics, wherein the sample, for example, may be a sample of a bodily fluid. The sample may be received in a sample vessel or container which is handled or processed in the laboratory system. The sample vessel may be handled by a sample vessel distribution system, for example, for locating the sample vessel in sample vessel carriers and for picking up the sample vessels from the sample vessel carriers. The laboratory system comprises one or more instruments or devices configured to process or handle the sample received in the sample vessel for at least one of sample pre-analytics and sample analytics.

In case of conducting maintenance for one or more of the instruments or devices, the laboratory system may be prevented from being operational, thereby, providing protection for a user applying maintenance task(s). For example, a cover of a cabinet receiving one or more instruments may need to be opened for maintenance. A safety look may be provided which automatically stops one or more instruments received within the cabinet laboratory system from operation such as moving in response to opening a door of the cabinet for maintenance.

Document US 9,316,662 B2 discloses an automated analyzer including a conveyance mechanism to convey a specimen such as a sample received in a sample vessel, an analysis portion to analyze the specimen, and a device cover to cover a movable mechanism including the conveyance mechanism. The automated analyzer is provided with an interlock mechanism and an interlock release mechanism. The interlock mechanism stops an operation of the movable mechanism when the device cover is opened. The interlock release mechanism disables all or part of the interlock mechanism. The interlock mechanism is enabled when a lever is in contact with a safety switch. The interlock mechanism is disabled or partially disabled when the lever is not in contact with the safety switch. This enables to prevent a user from inadvertently touching the movable mechanism including a hazard region during analysis of the automated analyzer or a maintenance task. Only some specific maintenance task can be performed with the device cover opened.

Document US 2015 / 0105878 A1 discloses methods and apparatus to provide a role-based user interface. A system includes a display device to depict a user interface. The system also includes a processor. The processor is to receive object information for an object in a process control system during a session, determine a user role based on the session, determine whether the object information is qualifying information based on the user role, and display the object information via the user interface when the object information is qualifying information.

Document US 2019 / 0012479 A1 discloses systems, devices, methods for controlling access to a medical device. A system includes an access card configured to wirelessly transmit user identification information and a medical device configured to receive the user identification information from the access card, and enable access to one or more components of the medical device based on permission information associated with the received user identification information. A medical device includes a plurality of device components; a data receiver configured to wirelessly receive user identification information; a memory configured to store permission information in association with the user identification information; and a processor configured to retrieve the permission information from the memory in response to receiving the user identification information from the data receiver, and enable access to the one or more device components based on the retrieved permission information.

Document EP 2 549 277 A2 refers to an analyzing system which includes an analyzer and a server computer in communication with the analyzer via a network. The analyzer includes a first controller that is configured to communicate, via a display, instructions to an operator of the analyzer when a predetermined event occurs in the analyzer. The instructions request confirmation that the operator received training. The first controller is further configured to receive an indication of whether the operator has completed the training, and, when the indication indicates that the operator has completed the training, request, from the server, a confirmation that the operator has completed the training. The server computer is configured to receive the confirmation request from the analyzer, determine a training status of the operator, and communicate the training status to the analyzer. The first controller prevents measurement of a sample if the operator has not completed the training.

Document US 8 745 756 B2 refers to a device management system which makes it possible to change and reset device-related operation rights assigned to users. User's operation rights with respect to a device are set, and a system policy is set which is used to restrict the operation rights with respect to the device and is to be applied to a plurality of specific users. It is determined whether or not the system policy is to be applied. If it is determined that the system policy is to be applied, the user's operation rights with respect to the device are restricted based on the system policy irrespective of whether or not operation rights have been set.

### Summary

It is an object to provide a method for performing a maintenance task in a laboratory system configured for at least one of sample pre-analytics and sample analytics and a laboratory systems which allow for improved controlling of maintenance applied to the laboratory system.

For solving the object, a method for performing a maintenance task in a laboratory system configured for at least one of sample pre-analytics and sample analytics according to claim 1 is provided. Further, a laboratory system configured for at least one of sample pre-analytics and sample analytics according to claim 13 is provided. Further embodiments are disclosed in the dependent claims.

According to one aspect, a method for for performing a maintenance task in a laboratory system configured for at least one of sample pre-analytics and sample analytics is provided, the method comprising: providing first permission data in a memory accessible by a maintenance access control device assigned to a laboratory system configured for at least one of sample pre-analytics and sample analytics, the first permission data being indicative of a plurality of user-specific maintenance modes applicable in the laboratory system, wherein each of the plurality of user-specific maintenance modes is assigned to at least one user and indicating one or more maintenance tasks permitted to be conducted by the at least one user in the user-specific maintenance mode; receiving user identification data indicative of a user in the maintenance access control device; receiving user selection data indicative of a user selection of a first maintenance mode from the plurality of user-specific maintenance modes in the maintenance access control device; determining whether the first maintenance mode is assigned to the user by the maintenance access control device; providing access control data in the maintenance access control device indicative of one of permitting the user to conduct the first maintenance mode, if is determined that the first maintenance mode is assigned to the user; and not permitting the user to conduct the first maintenance mode, if is determined that the first maintenance mode is not assigned to the user; and controlling maintenance operation of the laboratory system according to the access control data.

According to a further aspect, a laboratory system configured for at least one of sample pre-analytics and sample analytics is provided, the system comprising: one or more instruments configured to be operated for at least one of sample pre-analytics and sample analytics, a maintenance access control device, and a memory configured to store data accessible by the maintenance access control device. The laboratory system is configured to: provide first permission data in the memory, the first permission data being indicative of a plurality of user-specific maintenance modes applicable in the laboratory system, wherein each of the plurality of user-specific maintenance modes is assigned to at least one user and indicating one or more maintenance tasks permitted to be conducted by the at least one user in the user-specific maintenance mode; receive user identification data indicative of a user in the maintenance access control device; receive user selection data indicative of a user selection of a first maintenance mode from the plurality of user-specific maintenance modes in the maintenance access control device; determine whether the first maintenance mode is assigned to the user by the maintenance access control device; provide access control data in the maintenance access control device indicative of one of permitting the user to conduct the first maintenance mode, if is determined that the first maintenance mode is assigned to the user, and not permitting the user to conduct the first maintenance mode, if is determined that the first maintenance mode is not assigned to the user; and control maintenance operation of the laboratory system according to the access control data.

The method and the laboratory system provide for user-specific maintenance to be applied to instruments or devices of the laboratory system which, for example, may be an in-vitro diagnostic system for determining a sample of a bodily fluid. Prior to performing or conducting some maintenance mode comprising one or more maintenance tasks, the user who wants to apply such maintenance provides the user identification data for user identification. In response to receiving user selection data indicative of a user selection of a first maintenance mode from the plurality of user-specific maintenance modes, it is determined whether the first maintenance mode is assigned to the user, such assignment indicating that the user is allowed or permitted to conduct or perform maintenance task(s) assigned to the first maintenance mode. If it is determined that the user is allowed to perform the first maintenance mode, since the first maintenance mode is assigned to the user, such permit of conducting the first maintenance mode is indicated by the access control data. Conversely, if it is determined that the first maintenance mode is not assigned to the user providing the user identification data, the access control data will be indicative of not permitting the user to conduct or perform the first maintenance mode. Following, in dependence on the access control data indicating the user being allowed or not allowed to conduct the first maintenance mode, operation of the laboratory system is controlled. Thereby, maintenance applied to the laboratory system can be conducted user-specific. For example, depending on a level of skills of the user different level of maintenance work may be allowed for the different users.

It may be provided that one and the same maintenance mode is assigned to a plurality of different users such as service workers. Alternatively, one or more maintenance modes may be assigned to only a single user. Each user is only permitted or allowed to conduct or perform a maintenance mode which, according to the permission data, is assigned to such user.

Maintenance access control is implemented, at least in part, by the maintenance access control device assigned to the laboratory system. It may be implemented by at least one of one or more software components or applications and one or more hardware components such as one or more data processors configured to control access to the different user-specific maintenance modes. The maintenance access control device in part may be provided within the laboratory system itself or separated from the laboratory system, but operatively connected to the laboratory system, for example, to a control device of the laboratory system configured to control operation of the instruments or devices of the laboratory system. Data transmission between the maintenance access control device and the laboratory system may be conducted by at least one of wireless data communication and wired data communication.

The controlling of operation may further comprise preventing the laboratory system from conducting the first maintenance mode, if the access control data indicate the user not being permitted to conduct the first maintenance mode. The laboratory system is blocked form performing any maintenance task of the first maintenance mode, since it has been determined that the user identified by the user identification data is not allowed or permitted to perform the first maintenance mode. With respect to such example or in other examples, access control information indicative of the access control data may be outputted through an output device connected to the maintenance access control device for user information. The user will be informed whether be allowed to apply the first maintenance mode requested or not.

The method may further comprise the following: receiving first task input data indicative of a first maintenance task in the maintenance access control device; determining whether the first maintenance task is assigned to the first maintenance mode for which the user was determined to have permission by the maintenance access control device; providing first maintenance task control data in the maintenance access control device indicative of one of permitting the user to conduct the first maintenance task, if is determined that the first maintenance task is assigned to the first maintenance mode, and not permitting the user to conduct the first maintenance task, if is determined that the first maintenance task is not assigned to the first maintenance mode; and controlling operation of the laboratory system according to the first maintenance task control data. According to such example, after it has been determined that the first maintenance mode is assigned to the user, for one or more first maintenance tasks requested by the user it will be checked whether such maintenance task is actually permitted or allowed according to the first maintenance mode. If it is determined that the user has requested a maintenance task not being assigned to the first maintenance mode, the user will not be permitted to conduct such maintenance task. Thus, in the process of applying some maintenance in the laboratory system by the user, both the first maintenance mode and the one or more first maintenance tasks are checked whether it is allowed or not. Specific control of maintenance work to the laboratory system can be conducted.

The controlling of operation may further comprise preventing the laboratory system from conducting the first maintenance task, if the first maintenance task control data indicate the user not being permitted to conduct the first maintenance task. If it is determined that the first maintenance task is not assigned to the first maintenance mode permitted for the user, the laboratory system is blocked from performing such first maintenance task. The user will not be allowed or permitted to perform such first maintenance task, since it is not part of the first maintenance mode which was found to be assigned to the user. Again, the user may be informed by outputting user information indicative of the first maintenance task being permitted or not.

The receiving of user selection data may further comprise the following: outputting a user menu by an output device of a user interface such as a graphical user interface assigned to the laboratory system, the user menu being indicative of at least a subset of user-specific maintenance modes from the plurality of user-specific maintenance modes; and receiving user mode input data in an input device of the user interface, the user input data comprising the user selection data. By means of the user menu the user is presented at least the subset of user-specific maintenance modes, thereby, providing the user with the option to select at least one of the user-specific maintenance modes.

The outputting of the user menu may further comprise, in the maintenance access control device, determining the subset of user-specific maintenance modes from the plurality of user-specific maintenance modes in response to receiving a user request input for maintenance in the user interface.

The determining of the subset of user-specific maintenance modes may further comprise the following: determining, based on the user identification data indicative of the user, one or more user-specific maintenance modes from the plurality of user-specific maintenance modes, the one or more user-specific maintenance modes being assigned to the user; and providing subset of user-specific maintenance modes one or more user-specific maintenance modes assigned to the user. According to an example, the subset may be provided to exclusively comprise user-specific maintenance modes which, according to the permission data, are registered to be assigned to the user identified by the user identification data.

If the user wants to request access to some additional user-specific maintenance mode, the user may request presentation of additional user-specific maintenance modes in the menu, the additional user-specific maintenance modes not (yet) being assigned to the user. Following, the user may register one or more of the additional user-specific maintenance modes for himself. In an example, the subset of user-specific maintenance modes may be limited to only the one or more user-specific maintenance modes which, according to the permission data, are assigned to the user identified.

The providing of permission data may further comprise the following: receiving new mode input data indicative of one or more maintenance tasks applicable in the laboratory system; generating a new user-specific maintenance mode having assigned the one or more maintenance tasks indicated by the mode input data; assigning the new user-specific maintenance mode to the user; and storing second permission data indicative of the new user-specific maintenance mode assigned to the user. This will provide the user with the opportunity to have one or more new user-specific maintenance modes defined or established and assigned to such user.

The receiving of the new mode input data may further comprise: outputting a maintenance task menu by the output device, the maintenance task menu being indicative of a plurality of maintenance tasks applicable in the laboratory system; and receiving user task input data in the input device, the user task input data indicative of one or more maintenance tasks for the new user-specific maintenance mode. The maintenance task menu provides the plurality of maintenance tasks which may be selected by the user for generating the new user-specific maintenance mode. The plurality of maintenance task presented in the maintenance task menu may be a pre-selection of maintenance tasks in dependence on the user identified. For example, if the user is identified as a software engineer one or more software-related maintenance tasks may be presented only or as preferred maintenance task. Similarly, if the user is identified as being hardware specialist, one or more maintenance tasks related to hardware components may be presented in the maintenance task menu.

The storing of the second permission data may further comprise one of providing the new user-specific maintenance mode as an additional user-specific maintenance mode; and substituting a selected user-specific maintenance mode indicated by the first permission data by the new user-specific maintenance mode. The new user-specific maintenance mode may establish a maintenance mode in addition to existing maintenance modes assigned to the user. Alternatively, an existing user-specific maintenance mode may be substituted by the new user-specific maintenance mode, for example, if the new user-specific maintenance mode refers to only some amendment of the existing user-specific maintenance mode. One or more maintenance task may be amended because of such substitution.

The controlling of (maintenance) operation of the laboratory system may further comprise the following: receiving user maintenance start input prior to permitting the first maintenance mode performed in the laboratory system; and receiving user maintenance end input prior to permitting the first maintenance mode performed in the laboratory system after the first maintenance mode is completed. The user maintenance start input and the user maintenance end input, for example, may be provided by a mechanical lock activated / deactivated by the user. A key lock device may be provided for detecting the user maintenance input. The user-specific maintenance mode selected and permitted may only be started after receiving the user maintenance start input, for example, by the user operating the mechanical lock. Similarly, no further maintenance task may be permitted or allowed after receiving the user maintenance end input, for example, by activating the mechanical lock again. Thus, the user maintenance start / end input provide for additional control with respect to actually performing the one or more maintenance tasks of the user-specific maintenance mode.

The laboratory system may comprise one or more instruments configured to be operated for at least one of sample pre-analytics and sample analytics. The one or more maintenance tasks performed in the first maintenance mode may be selected from the following group: disconnecting one or more of the instruments from electric power supply; one or more of the instruments receiving power supply; one or more device being operable; one or more device being operable in a maintenance mode of operation different from normal mode of operation applied in ate least one of sample pre-analytics and sample analytics; preventing one or more of the instruments from operation.

With respect to the one or more instruments, the maintenance tasks performed in the first maintenance mode may refer to maintaining or checking at least one of a compressed air manometer switch, a compressed air filter, and a pressure control valve. Emptying of condensation may be conducted. Alternatively or in addition, in a maintenance task at least one of recalibration, realignment, and cleaning my be applied. For example, a laser liquid level detection unit may be recalibrated. Further, a laser diode or photodiode may be realigned and / or cleaned. Cleaning may be applied in addition or alternatively for at least one of a bar code reader, a bar code printer, a camera, and a pipetting nozzle. In an example, a maintenance task may be performed for recalibrating a camera view and / or an entry drive system, e.g., a motor controller and / or a drive-belt may be readjusted and re-teached. In an example, a motor drive encoder adjusted to a coordinate system of a gripper system for handling sample containers may be re-adjusted.

The maintenance tasks performed in the first maintenance mode may comprise reading out an error log comprising error log data indicative of, for example, operation errors detected while operating the laboratory system.

The embodiments outlined above with respect to the method for performing the maintenance task in the laboratory system may be applied to the laboratory system configured for at least one of sample pre-analytics and sample analytics *mutatis mutandis.*

### Description of further embodiments

Following, further embodiments are disclosed by referring to figures. In the figures, show:
- Fig. 1: a schematic representation of a cabinet of a laboratory system configured for at least one of sample pre-analytics and sample analytics;
- Fig. 2: another schematic representation of the cabinet in Fig. 1;
- Fig. 3: a schematic representation of a cabinet drawer loaded with sample vessels carriers;
- Fig. 4: a schematic representation of the cabinet drawer from Fig. 3 without sample vessels carriers;
- Fig. 5: a schematic representation of an arrangement for maintenance access control assigned to a laboratory system;
- Fig. 6: a schematic representation of a user menu presented to a user through a user interface; and
- Fig. 7: a schematic representation of a maintenance task menu presented to a user through the user interface.

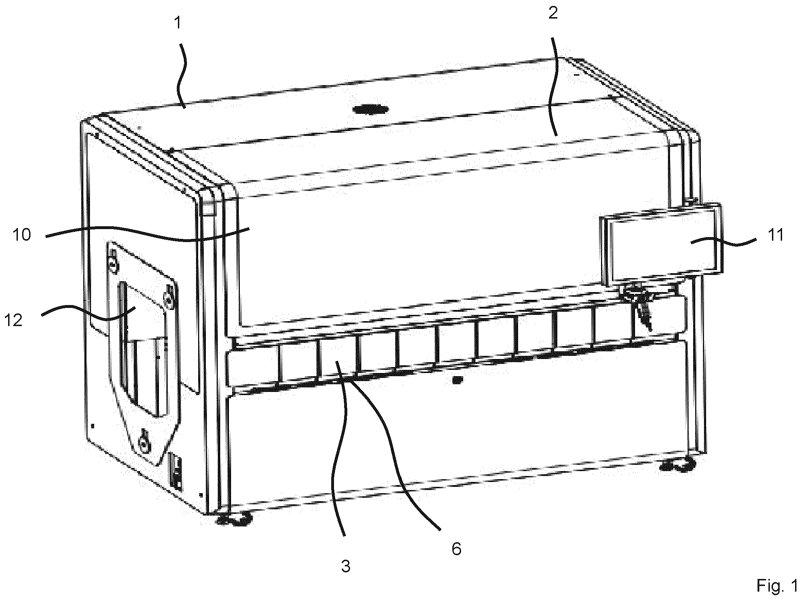

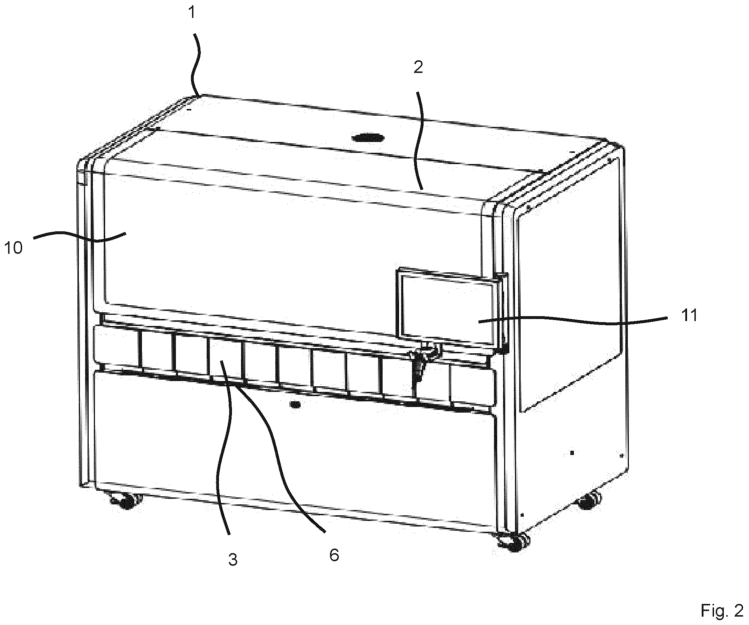

Fig.1 and 2 show a schematic representation of a cabinet 1 of a laboratory system configured for at least one of sample pre-analytics and sample analytics. Within the cabinet 1 an interior space 2 is provided which may also be referred to as working space.

The laboratory system may be implementing an in-vitro diagnostic system. The laboratory system may be provided with a plurality of cabinets receiving one or more instruments or devices configured to conduct at least one of sample pre-analytics and sample analytics for a sample such as a sample of a bodily fluid.

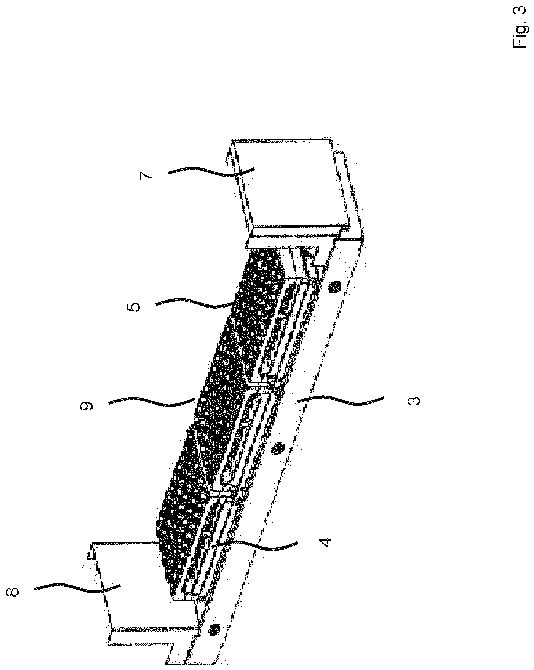

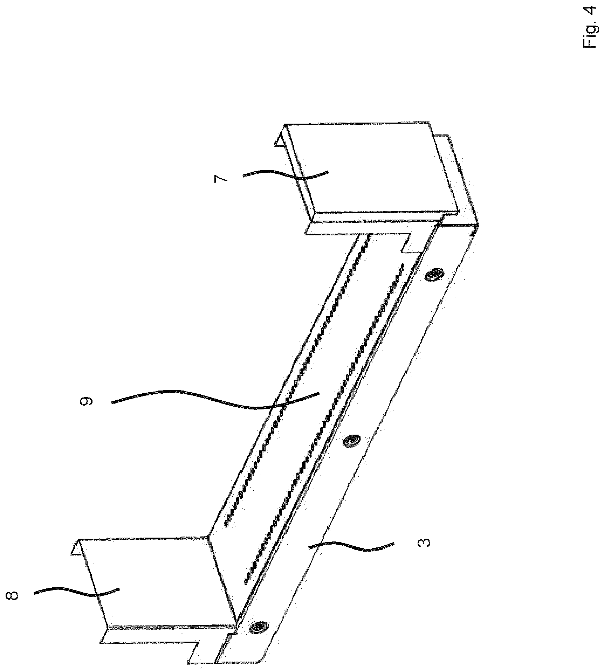

Fig. 3 and 4 show a schematic representation of a cabinet drawer 3 loaded with sample vessel carriers 4 receiving sample vessels 5, and a schematic representation of the cabinet drawer 3 without sample vessel carriers 4, respectively. The cabinet drawer 3 is to be received in a drawer opening 6 provided in the cabinet 1 (cf. Fig. 1 and 2). The cabinet drawer 3 comprises a front panel 7 and a back panel 8 providing a front cover and a back cover, respectively, for a space 9 of the cabinet drawer 3 for receiving the sample vessel carriers 4.

Referring to Fig. 1 and 2, if the cabinet drawer 3 is in a closed position, the drawer opening 6 is (fully) covered or closed by the front panel 7. Similarly, if the cabinet drawer 3 is in a fully opened position (not shown), the drawer opening 6 is (fully) covered or closed by the back panel 8. Thus, in case the cabinet drawer 3 is located in one of the closed position and the fully opened position, a user operating or interacting with the laboratory sample vessel distribution system does not have access to the interior space 3 within the cabinet 1 through the drawer opening 6 (accidently or purposefully).

Contrary, in case the cabinet drawer 3 is in an opened position in which the cabinet drawer 3 is not fully, but partially pulled out, the drawer opening 6 is not covered or closed by either the front panel 7 or the back panel 8. The drawer opening 6 being at least in part open will give the user access to the interior space 2.

For the cabinet 1, there is a plurality of separated drawer openings 6 each receiving one or more cabinet drawers 3.

The cabinet 1 is provided with a cabinet door 10 relocatable between a closed position (shown in Fig. 1 and 2) and an open position (not shown) by lifting up the cabinet door 10. If the cabinet door 10 is in the open positon, the user has access to the interior space 2. In the closed position the interior space 2 is fully covered.

A display panel 11 provides for a user interface such as a graphical user interface configured to receive user input and output user information. For receiving user input the display panel 11 may be provided with a touch sensitive screen.

In the interior space 2 the sample vessel carriers 4 may be picked up from the cabinet drawer 3 if the cabinet drawer 3 is in the closed position (see Fig. 1 and 2), for example, for placing the sample vessel carriers 4 on a transport belt or robot configured to transport (by movement) the sample vessel carriers 4 to some working station (not shown) inside or outside of the cabinet 1. Transporting the sample vessel carriers 4 outside the cabinet may be conducted through a wall opening 12.

Further, in the interior space 2 the sample vessels 5 may be picked up from the sample vessel carriers 4 and relocated. For example, a sample vessel 5 may be moved to some working station provided with one or more instruments or devices of the laboratory system in a line of sample processing (not shown).

For processing, the sample vessels 5 receiving the sample of the bodily fluid to be analyzed may be moved in the line of processing by one or more actuators provided by other instruments or devices of the laboratory system. The one or more actuators may comprise, for example, at least one of a sample vessel gripper, a conveyor device, and a gantry robot. Such actuators of different type applied in the laboratory system are known as such. For example, the gantry robot allows for linear movement along three principle axis. Gantry robots may also be referred to as cartesian coordinate robots and are known as such.

In the line of processing the sample vessels 5 containing a sample to be determined are moved to a plurality of working stations. The different working stations may be provided for applying different steps in the process of determining and / or processing the sample received in the sample vessels or containers 5 for at least one of sample pre-analytics and sample analytics. For example, in a first working station the sample received in one of the sample vessels 5 may be prepared. For preparation, the sample vessel may be provided to a vibrating device for vibrating the sample vessel, thereby, supporting mixing of sample components. In a second working station which is different for the first working station, the sample received in the sample vessel may be analyzed or determined. In an embodiment, an optical measurement such as transmission and / or absorbance measurement may be applied to the sample received in the sample vessel by an optical sample measurement module (not shown). Between the different working stations or locations the sample vessels 5 may be processed by moving the sample vessels 5 by means of the actuator(s).

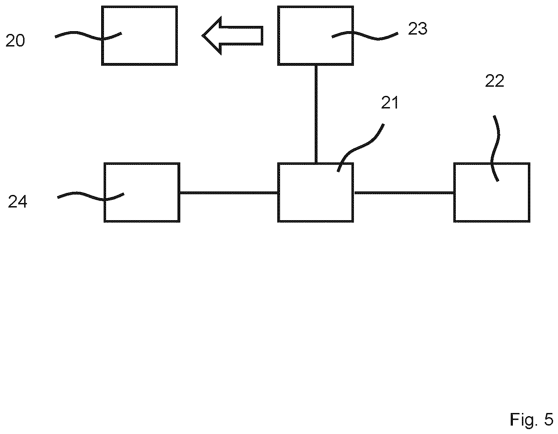

Fig. 5 shows a schematic representation of an arrangement for maintenance access control assigned to a laboratory system 20 which may comprise one or more cabinets like the cabinet 1 and is configured for at least one of sample pre-analytics and sample analytics. A maintenance access control device 21 and a memory device 22 are provided. The memory device 22 is configured to store electronic data. It is accessible by the maintenance access control device 21.

The maintenance access control device 21 is connected to an input device 24 which may be provided with or by the display panel 11 of the user interface. Further, the maintenance access control device 21 is connected to a control device 23 of the laboratory system 20 which is configured to control operation of the instruments or devices of the laboratory system 20, for example, instruments or devices provided with the one or more working stations, specifically operation related to maintenance. However, operation control provided by the control device 23, in another example, may not be limited to operation while maintenance is applied, but may also refer to operation control while a sample analysis and / or sample pre-analytics is performed.

In an operation for controlling maintenance work to be applied to the laboratory system 20, first permission data are provided in the memory device 22. The first permission data are indicative of a plurality of user-specific maintenance modes applicable in the laboratory system 20 to one or more of the instruments or devices. User-specific maintenance modes refer to one or more maintenance tasks, for example, software and / or hardware maintenance applied for one or more of the instruments or devices of the laboratory system 20.

Each of the user-specific maintenance modes is assigned to at least one user, for example, a service staff member. Each user-specific maintenance mode is assigned one or more maintenance tasks which may be applied to one or more instruments or devices of the laboratory system 20 in the course of a maintenance mode. The maintenance task may refer to at least one of the following: disconnecting one or more of the instruments from electric power supply; one or more of the instruments receiving power supply; one or more devices being operable; one or more devices being operable in a maintenance mode of operation different from normal mode of operation applied in at least one of sample pre-analytics and sample analytics; and preventing one or more of the instruments from operation.

If a user wants to conduct maintenance with respect to the laboratory system 20, the user has to provide user identification data which is received in the maintenance access control device 21, for example, through the input device 24. For example, the user may provide an ID card (ID - identification) or a badge to a card reader device connected to the input device 24.

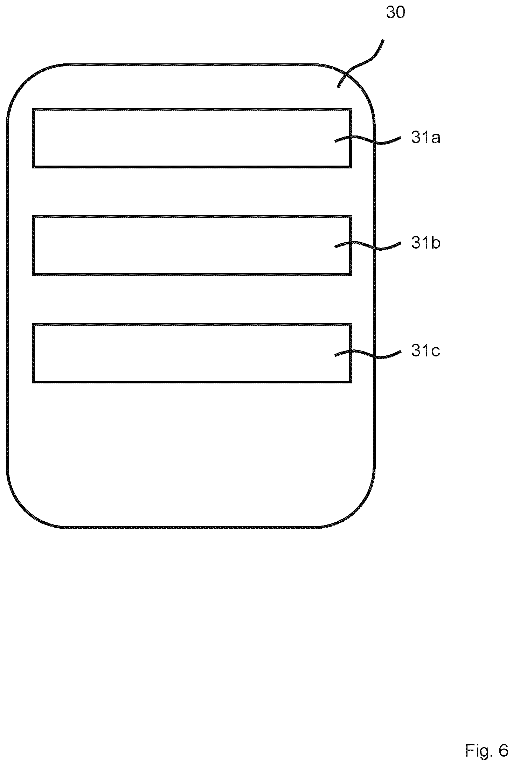

Further, a request for performing maintenance is received in the maintenance access control device 21 from the user. In response, according to an example shown in Fig. 6, a user menu 30 is presented on the display device 11, the user menu 30 comprising a plurality of user-specific maintenance modes 31a, ..., 31c which are available for application in the laboratory system 20.

In response to the user selecting one of the user-specific maintenance modes 31a, ..., 31c, in the maintenance access control device 21 it is determined whether such user-specific maintenance mode selected by the user is assigned to the user according to the first permission data. If it is determined that the first maintenance mode selected by the user is assigned to the user, access control data are provided in the maintenance access control device 21, the access control data being indicative of a permission for the user to conduct the maintenance mode selected. If it is determined that the maintenance mode selected by the user is not assigned to such user, the access control data will be indicative of not permitting the user to perform the maintenance mode selected.

Based on such access control data operation of the laboratory system 20 is controlled by the control device 23. If the user is not permitted to conduct the maintenance mode selected, the laboratory system 20 is prevented from conducting the maintenance mode selected by the user. Otherwise, the user can perform maintenance task assigned to the maintenance mode selected.

Maintenance access control is implemented, at least in part, by the maintenance access control device 21 assigned to the laboratory system 20. The maintenance access control device 21 may be implemented by at least one of one or more software components or applications and one or more hardware components such as one or more data processors configured to control access to the different user-specific maintenance modes. The maintenance access control device 21 in part may be provided within the laboratory system 20 itself or separated from the laboratory system 20, but operatively connected to the laboratory system 20, for example, to the control device 23 of the laboratory system 20 configured to control operation of the instruments or devices of the laboratory system 20. Data transmission between the maintenance access control device 21 and the laboratory system 20 may be conducted by at least one of wireless data communication and wired data communication.

The user (maintenance) menu 30 of Fig. 6 is presented on the display device 11. The user menu 30 comprises the plurality of maintenance modes 31a, ..., 31c applicable in the laboratory system 20. The user may select one of the maintenance modes 31a, ..., 31c to be performed. Following, it is determined whether the user is allowed to perform such selected as described above.

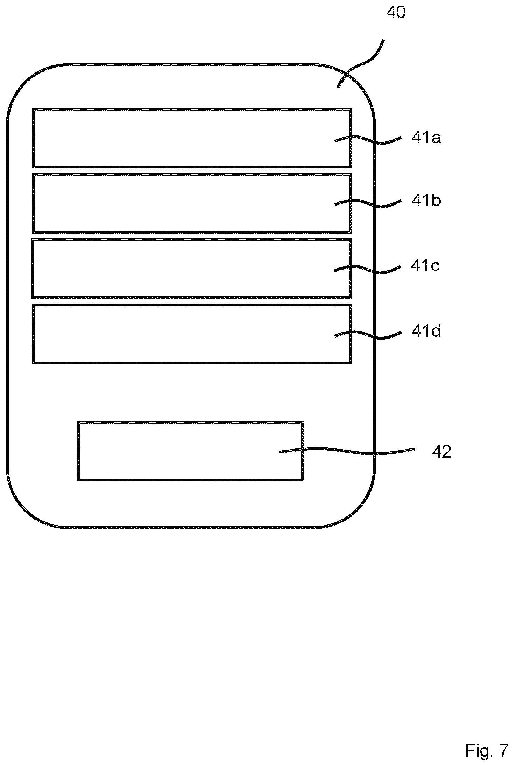

In another example, referring to Fig. 7, the user may be presented a maintenance task menu 40 being indicative of a plurality of maintenance tasks 41a, ..., 41d applicable in the laboratory system 20 while conducting a maintenance mode such as one of the maintenance modes 31a, ..., 31c. The user can select one or more of the maintenance tasks 41a, ..., 41d and create a new user-specific maintenance mode comprising the maintenance tasks selected by the user by selecting a field 42 for creating new user-specific maintenance mode. In response to generating the new user-specific maintenance mode, such new user-specific maintenance mode may be added to the first permission data provided in the memory device 21. Alternatively, the new user-specific maintenance mode may be substituting an existing user-specific maintenance mode assigned to the user.

It may be provided that a user-specific maintenance mode selected by the user can only be started after the user has activated a mechanical lock or a key lock provided in the laboratory system 20, for example, a lock activated in response to opening / closing the cabinet drawer 3. Similarly, performing additional maintenance task may be prevented after the user has again activated the mechanical lock indicating the end of the maintenance mode.

## Claims

1. A method for performing a maintenance task in a laboratory system configured for at least one of sample pre-analytics and sample analytics, comprising
- providing first permission data in a memory (22) accessible by a maintenance access control device (21) assigned to a laboratory system (20) configured for at least one of sample pre-analytics and sample analytics, the first permission data being indicative of a plurality of user-specific maintenance modes (31a, ..., 31c) applicable in the laboratory system (20), wherein each of the plurality of user-specific maintenance modes (31a, ..., 31c) is assigned to at least one user and indicating one or more maintenance tasks permitted to be conducted by the at least one user in the user-specific maintenance mode;
- receiving user identification data indicative of a user in the maintenance access control device (21);
- receiving user selection data indicative of a user selection of a first maintenance mode from the plurality of user-specific maintenance modes (31a, ..., 31c) in the maintenance access control device (21);
- determining whether the first maintenance mode is assigned to the user by the maintenance access control device (21);
- providing access control data in the maintenance access control device (21) indicative of one of
- permitting the user to conduct the first maintenance mode, if is determined that the first maintenance mode is assigned to the user; and
- not permitting the user to conduct the first maintenance mode, if is determined that the first maintenance mode is not assigned to the user; and
- controlling operation of the laboratory system (20) according to the access control data.

2. Method of claim 1, wherein the controlling of operation further comprises preventing the laboratory system (20) from conducting the first maintenance mode, if the access control data indicate the user not being permitted to conduct the first maintenance mode.

3. Method of claim 1 or 2, further comprising
- receiving first task input data indicative of a first maintenance task in the maintenance access control device (21);
- determining whether the first maintenance task is assigned to the first maintenance mode for which the user was determined to have permission by the maintenance access control device (21);
- providing first maintenance task control data in the maintenance access control device (21) indicative of one of
- permitting the user to conduct the first maintenance task, if is determined that the first maintenance task is assigned to the first maintenance mode; and
- not permitting the user to conduct the first maintenance task, if is determined that the first maintenance task is not assigned to the first maintenance mode; and
- controlling operation of the laboratory system (20) according to the first maintenance task control data.

4. Method of claim 3, wherein the controlling of operation further comprises preventing the laboratory system (20) from conducting the first maintenance task, if the first maintenance task control data indicate the user not being permitted to conduct the first maintenance task.

5. Method of at least one of the preceding claims, wherein the receiving of user selection data further comprises
- outputting a user menu (30) by an output device (11) of a user interface assigned to the laboratory system (20), the user menu being indicative of at least a subset of user-specific maintenance modes from the plurality of user-specific maintenance modes (31a, ..., 31c); and
- receiving user mode input data in an input device (24) of the user interface, the user input data comprising the user selection data.

6. Method of claim 5, wherein the outputting of the user menu further comprises, in the maintenance access control device (21), determining the subset of user-specific maintenance modes from the plurality of user-specific maintenance modes (31a, ..., 31c) in response to receiving a user request input for maintenance in the user interface.

7. Method of claim 6, wherein the determining of the subset of user-specific maintenance modes further comprises
- determining, based on the user identification data indicative of the user, one or more user-specific maintenance modes from the plurality of user-specific maintenance modes (31a, ..., 31c), the one or more user-specific maintenance modes being assigned to the user; and
- providing subset of user-specific maintenance modes one or more user-specific maintenance modes assigned to the user.

8. Method of at least one of the preceding claims, wherein the providing of permission data further comprises
- receiving new mode input data indicative of one or more maintenance tasks applicable in the laboratory system (20);
- generating a new user-specific maintenance mode having assigned the one or more maintenance tasks indicated by the mode input data;
- assigning the new user-specific maintenance mode to the user; and
- storing second permission data indicative of the new user-specific maintenance mode assigned to the user.

9. Method of claim 8, wherein the receiving of the new mode input data further comprises
- outputting a maintenance task menu (40) by the output device, the maintenance task menu (40) being indicative of a plurality of maintenance tasks (41a, ..., 41d) applicable in the laboratory system (20); and
- receiving user task input data in the input device, the user task input data indicative of one or more maintenance tasks for the new user-specific maintenance mode.

10. Method of at least claim 8 or 9, wherein the storing of the second permission data further comprises one of
- providing the new user-specific maintenance mode as an additional user-specific maintenance mode; and
- substituting a selected user-specific maintenance mode indicated by the first permission data by the new user-specific maintenance mode.

11. Method of at least one of the preceding claims, wherein the controlling of operation of the laboratory system (20) further comprises
- receiving user maintenance start input prior to permitting the first maintenance mode performed in the laboratory system (20); and
- receiving user maintenance end input prior to permitting the first maintenance mode performed in the laboratory system (20) after the first maintenance mode is completed.

12. Method of at least one of the preceding claims, wherein
- the laboratory system (20) comprises one or more instruments configured to be operated for at least one of sample pre-analytics and sample analytics; and
- the one or more maintenance tasks performed in the first maintenance mode are selected from the following group:
- disconnecting one or more of the instruments from electric power supply;
- one or more of the instruments receiving power supply;
- one or more device being operable;
- one or more device being operable in a maintenance mode of operation different from normal mode of operation applied in ate least one of sample pre-analytics and sample analytics;
- preventing one or more of the instruments from operation;

13. A laboratory system (20) configured for at least one of sample pre-analytics and sample analytics, comprising
- one or more instruments configured to be operated for at least one of sample pre-analytics and sample analytics;
- a maintenance access control device (21); and
- a memory (22) configured to store data accessible by the maintenance access control device (21);
wherein the laboratory system (20) is configured to
- provide first permission data in the memory (22), the first permission data being indicative of a plurality of user-specific maintenance modes (31a, ..., 31c) applicable in the laboratory system (20), wherein each of the plurality of user-specific maintenance modes (31a, ..., 31c) is assigned to at least one user and indicating one or more maintenance tasks permitted to be conducted by the at least one user in the user-specific maintenance mode;
- receive user identification data indicative of a user in the maintenance access control device (21);
- receive user selection data indicative of a user selection of a first maintenance mode from the plurality of user-specific maintenance modes (31a, ..., 31c) in the maintenance access control device (21);
- determining whether the first maintenance mode is assigned to the user by the maintenance access control device (21);
- provide access control data in the maintenance access control device (21) indicative of one of
- permitting the user to conduct the first maintenance mode, if is determined that the first maintenance mode is assigned to the user, and
- not permitting the user to conduct the first maintenance mode, if is determined that the first maintenance mode is not assigned to the user; and
- control maintenance operation of the laboratory system (20) according to the access control data.
